# EUROPEAN PATENT SPECIFICATION

(11) **EP 1 691 813 B1**
(45) Date of publication and mention of the grant of the patent: **19.03.2008**
(21) Application number: 04797152.8
(22) Date of filing: 25.11.2004
(51) Int. Cl.: A61K 31/52, B32B 27/32, B32B 27/36, A61J 1/00

(54) **READY-FOR-USE INJECTABLE SOLUTION OF 9-((1,3-DIHYDROXYPROPAN-2-ILOXY)METHYL)-2-AMINE-1H-PURIN-6(9H)-ONE, STERILE, STABLE; CLOSED SYSTEM FOR PACKING THE SOLUTION, PROCESS FOR ELIMINATING ALKALINE RESIDUALS OF 9-((1,3-DIHYDROXYPROPAN-2-ILOXY)METHYL)-2-AMINE-1H-PURIN-6(9H)-ONE CRYSTALS;**
GEBRAUCHSFERTIGE, STERILE, STABILE INJEKTIONSLÖSUNG VON 9-((1,3-DIHYDROXYPROPAN-2-ILOXY)METHYL)-2-AMIN-1H-PURIN-6(9H)-ON; GESCHLOSSENES SYSTEM ZUR VERPACKUNG DER LÖSUNG, VERFAHREN ZUR ELIMINIERUNG VON ALKALISCHEN RESTEN VON 9-((1,3-DIHYDROXYPROPAN-2-ILOXY)METHYL)-2-AMIN-1H-PURIN-6(9H)-ON-KRISTALLEN,
SOLUTION INJECTABLE PRETE A L'EMPLOI, STERILE, STABLE, DE 9-((1,3-DIHYDROXYPROPAN-2-ILOXY)METHYL)-2-AMINE-1H-PURIN-6(9H)-ONE; SYSTEME FERME PERMETTANT DE CONDITIONNER LA SOLUTION, PROCEDE D'ELIMINATION DES RESIDUS ALCALINS DES CRISTAUX DE 9-((1,3-DIHYDROXYPROPAN-2-ILOXY)METHYL)-2-AMINE-1H-PURIN-6(9H)-ONE

(30) Priority: 28.11.2003 BR PI0305339; 23.11.2004 BR PI0405798
(43) Date of publication of application: 23.08.2006
(73) Proprietor: Halex Istar Industria Farmaceutica Ltda., Goiania 74775-027 (BR); Labogen S/A Quimica Fina e Biotecnologia, 13347-330 Indaiatuba (BR)
(72) Inventor: PERILLO, Heno, 74120-050 Goiânia (BR); CAMPOS NETO, José, 13347-330 Indaiatuba (BR); CRUCELLI, Márcio, 13347-330 Indaiatuba (BR)
(74) Representative: Alves Moreira, Pedro
(86) International application number: PCT/BR2004/000235
(87) International publication number: WO 2005/051286

(56) References cited:
- EP-A1- 1 023 899
- EP-A2- 0 733 472
- CN-A- 1 403 084
- JP-A- 2003 073 395
- DATABASE HCAPLUS [Online] TAKASHI O. ET AL: 'Stability and physicochemical properties of ganciclovir.', XP003000814 Database accession no. (1998:251977) & KAGAKU RYOHO NO RYOIKI. vol. 14, no. 4, 1998, pages 670 - 672

## Description

### FIELD OF THE INVENTION

The present invention describes a sterile, stable and ready-for-use injectable solution comprising 9-((1,3-dihydroxypropan-2-iloxy)methyl)-2-amine-1H-purin-6(9H)-one in glucose 5% aqueous solution or sodium chloride 0.9% aqueous solution. It also describes a closed system used for packing the solution, which consists of a flexible bag manufactured with a tri-laminated material. The present invention also describes a process for eliminating the residual alkalinity that is present in 9-((1,3-dihydroxypropan-2-iloxy)methyl)-2-amine-1H-purin-6(9H)-one, so obtaining crystals that are free from alkaline residues and suitable for the direct preparation of the solution.

### BACKGROUND OF THE INVENTION

Gancyclovir (9-((1,3-Dihydroxy-2-propoxy)methyl)guanine or DHPG, or 2-Amine-1,9-((2-hydroxy-1-(hydroxymethyl) ethoxy)methyl)-6-H-purin-6-one, or 9-((1,3-dihydroxypropan-2-iloxy)methyl)-2-amine-1H-purin-6(9H)- one) is a synthetic drug derived from acyclovir, as described in patent US 4,355,032, showing to be active against most of herpes virus, with an activity of 100 times greater against cytomegalovirus. It was approved as a pharmaceutical drug in 1989, as an intravenous solution for treating retinitis in immunodepressed patients. Because its toxicity, it is only used for treating seriously immunodepressed patients, like AIDS infected and immunotransplanted individuals presenting serious disease related to cytomegalovirus infection.

In herpes simplex virus infected cells the mechanism of action is the same of Acyclovir. Cytomegalovirus does not have viral specific thymidine kinase, but the initial Ganciclovir phosphorylation is done by a phosphotransferase encoded by the virus gene.

Pretty much similar to Acyclovir - which is the nucleoside with the best therapeutic index among antiviral agents used for treating herpes simplex virus (HSV) infections type 1, type 2 and varicella zoster - in relation to the mechanism of action, initially it was used for treating immunodepressed patients infected by cytomegalovirus. It acts in two ways: by competitive inhibiting viral DNA-polymerase and by direct incorporation into viral DNA. It possess a broad spectrum activity including Epstein-Barr virus, cytomegalovirus, adenovirus, herpes zoster virus, and herpes virus types 1 and 2. Its efficacy, tolerance, and intraocular penetration was demonstrated by experimental studies of herpes keratitis in rabbits.

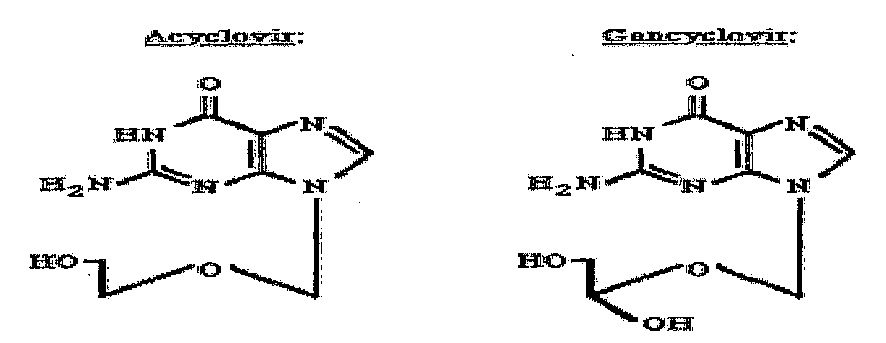

The 9-((1,3-dihydroxypropan-2-iloxy)methyl)-2-amine-1H-purin-6(9H)-one, as its sodium salt, is commercialized as a lyophilized powder. The lyophilized product is prepared by dissolving the active pharmaceutical ingredient (free acid form) in sodium hydroxide and it is submitted to sterile filtration, filling and subsequent lyophilization, so obtaining the lyophilized within the accepted technical specifications.

The manufacturer of the lyophilized powder recommends that preparations of 9-((1,3-dihydroxypropan-2-iloxy)methyl)-2-amine-1H-purin-6(9H)-one sodium salt in compatible infusion solutions, must be stored under refrigerating, but not under freezing. The 9-((1,3-dihydroxypropan-2-iloxy) methyl)-2-amine-1H-purin-6(9H)-one once reconstituted by using sterile water for injection and diluting with sodium chloride 0.9% solution in PVC bags, it is physically and chemically stable for 14 days when stored under refrigeration at 5°C. However, because the absence of preservative antimicrobial agents, it is recommended to be used within 24 hours (Reference: Lawrence A. Trissel - Handbook on Injectable Drugs 11th edition, pages 613 to 616).

In counterpart, the new formulation in a closed system disclosed in this application is sterile and stable for at least 24 months when stored at room temperature (20 to 30°C) .

According to Brazilian application PI 9803096 from LABOGEN S/A QUIMICA FINA E BIOTECNOLOGIA, the current marketed presentation of lyophilized 9-((1,3-dihydrohypropan-2-iloxy)methyl)-2-amine-1H-purin-6(9H)-one sodium salt can be improved in matter of process as well as the product itself, and this application, mentioned just as a reference from the state of the art, discloses the pharmaceutical active ingredient as alkaline salts of sodium and potassium, in an aqueous solution enclosed in ampoules with a concentration suitable for direct administration, and sterilized for assuring its chemical and microbiological properties. The resulting product is transferred into glass ampoules, sterilized by autoclaving and preserved within the useful definite conditions for the final client.

### THE CLOSED SYSTEM

The concept of closed system for parenteral solutions is based in the fact that there is no contact between the environment and the solution to be administered, by this way avoiding the microbiological contamination by air or by contact during the connection of the administration equipment.

Parenteral solutions may be packed in plastic flasks being designated as open systems, where there is no total protection against contamination. In this case, during the administration of the product to a patient vacuum develops, slowing the rate of the dropping solution. Besides, other drugs are added by withdrawing the device connected to the flask incurring in higher risks of contamination.

Packing 9-((1,3-dihydroxypropan-2-iloxy)methyl)-2-amine-1H-purin-6(9H)-one in closed systems with glucose or sodium chloride solution where not feasible until now because the instability of Ganciclovir sodium salt diluted in sodium chloride 0.9% and glucose 5% solutions, because an alkaline pH (about 11) is not compatible with the pH of those solutions (sodium chloride, Ringer lactate and glucose tolerate a maximum pH of 6).

Starting from this principle, the present invention proposes packing the 9-((1,3-dihydroxypropan-2-iloxy)methyl)-2-amine-1H-purin-6(9H)-one as its free acid form with glucose or sodium chloride solution in a closed system, thus avoiding the existing inconveniences found in the state of art, -including those observed in packed ampoules.

### THE INVENTION

In the production of 9-((1,3-dihydroxypropan-2-iloxy)methyl)-2-amine-1H-purin-6(9H)-one in glucose solution it was possible to notice the development of yellow coloration during the sterilization process and during accelerated stability studies, where the product was submitted to high temperatures, which indicated degradation. It was possible to reach a stabilized product by the technology of the present invention, by altering the kind of crystal from 9-((1,3-dihydroxypropan-2-iloxy)methyl)-2-amine-1H-purin-6(9H)-one molecule (Ganciclovir).

By this observation, the present invention concludes that the use of pre-diluted preparations in a closed system reduces medicines administration errors, reducing manipulation steps performed by nursing personnel, as well reducing contamination risks.

The product resulting from the present invention presents itself as a glucose or sodium chloride 0.9% solution with 9-((1,3-dihydroxypropan-2-iloxy)methyl)-2-amine-1H-purin-6(9H)-one, which can be packed in a flexible plastic bag (closed system).

As one of its objectives, the present invention describes a process for preparing the pharmaceutical active ingredient, 9-((1,3-dihydroxypropan-2-iloxy)methyl)-2-amine-1H-purin-6(9H)-one, in its elementary form (free acid form or non saline), without free alkaline residues resulting from the manufacturing process by its purification/crystallization.

The product of the present invention presents as its main characteristics the fact of being stable, being adequate to be stored in a closed system by a sterile plastic bag, being sterile, presenting a pH suitable for its storing in a closed system as a glucose or sodium chloride solution, which is the same pH of the solution, and being ready-for-use.

The stabilization of the final solution was possible because of the alteration of the kind of crystal from 9-((1,3-dihydroxypropan -2-iloxy) methyl) -2-amine-1H-purin-6(9H)-one, which can be achieved by the process for eliminating alkaline residues that are present in the crystals.

The altering in the active pharmaceutical ingredient crystal state resulting from the process, among them - shape, size, particle size, clearness, etc., characterizes another peculiar aspect of the active agent so prepared that differences itself from the product described in the state of art .

The Patent U.S. 4642346 discloses a novel anhydrous crystalline form of 9-((1,3-dihydroxy-2-propoxymethyl)guanine and a processes for its preparation. The major process involves only 3 steps of purification and no care is taken to eliminate alkaline residues from the crystals. Furthermore, another alternative process is claimed in which only a heating step (200-220 °C) is used to obtain said novel crystals from 9-((1,3-dihydroxy-2-propoxymethyl)guanine hydrate.

So, another objective of the present invention is the process for eliminating alkaline residues from 9-((1,3-dihydroxypropan-2-iloxy)methyl)-2-amine-1H-purin-6(9H)-one crystals and the final characteristics of the resulting crystals, as well as its purpose for preparing a pharmaceutical product to be stored in a closed system, the target of the invention.

The active pharmaceutical ingredient without free alkaline residues is essential for preparing one of the new pharmaceutical presentations of the product, in glucose solution, once the glucose, a starting material of the glucose solution with 9-((1,3-dihydroxypropan,2-iloxy) methyl) -2-amine-1H-purin-6 (9H) -one, reacts with alkalis forming furfural and methyl furfural, and these reacts with 9-((1,3-dihydroxypropan-2-iloxy)methyl)-2-amine-1H-purin-6(9H)-one present in the glucose solution generating further undesired substances, still under research.

During its purification/crystallization the 9-((1,3-dihydroxypropan-2-iloxy) methyl) -2-amine-1H-purin-6(9H)-one (free acid form), generates crystals with the inclusion of some parts per billion (PPB) of alkaline residues. These alkaline residues are responsible for glucose degradation and, therefore, provoke the degradation of 9-((1,3-dihydroxypropan-2-iloxy) methyl) -2-amine-1H-purin-6(9H)-one present in the glucose solution.

The process for preparing the active pharmaceutical ingredient crystals free from alkaline residues starts by preparing a suspension of 80 to 110g, preferably 100g of 9-((1,3-dihydroxypropan -2-iloxy) methyl) -2-amine-1H-purin-6(9H)-one (free acid form) in 0.9 to 1.1L, preferably in 1L of demineralized water. Next, it is added 13.5 to 16.5g, preferably 15g, of inorganic bases like potassium hydroxide, lithium hydroxide, sodium hydroxide (caustic soda), preferably sodium hydroxide, until reaching a pH from 10.5 to 12.5, when all solids dissolve forming a solution. When using caustic soda the preferential amount is about 15g and the preferential pH is 11.5. Next, the solution is heated to a temperature ranging from 75° to 90°C, preferably 85°C, and 5.4 to 6.6g, preferably 6g, of acids like fuming hydrochloric acid, hydrofluoric acid, acetic acid, citric acid are added until reaching a pH ranging from 4.5 to 5.5, preferably 4.5. Then the solution is cooled to a temperature ranging from 5° to 7°C, preferably 5°C, crystallizing the 9-((1,3-dihydroxypropan -2-iloxy) methyl)-2-amine-1H-purin-6(9H)-one (free acid form). After 25 to 40 minutes, preferably 30 minutes, under the temperature described above, the solid is filtered and washed with organic solvents like isopropanol, acetone, ethanol, methanol, preferably isopropanol. After the filtration, the resulting solid of 9-((1,3-dihydroxypropan-2-iloxy)methyl)-2-amine-1H-purin-6(9H)-one (free acid form) is suspended in isopropanol and intensely refluxed for 3 to 4 hours, preferably 4 hours. Then the resulting suspension is cooled to room temperature, between 20° to 30°C, preferably 25°C, and it is immediately filtered. The solid of 9-((1,3-dihydroxypropan-2-iloxy)methyl)-2-amine-1H-purin-6(9H)-one (free acid form) is dried in vacuum oven for 3 to 5 hours, preferably 4 hours at a temperature ranging from 60° to 80°C, preferably 70°C, so obtaining 90.4g to 100.4g, preferably 95,4g of the dried product.

More detailed, the process of the present invention starts in a glass reactor coupled with a condenser apparatus wherein the 9-((1,3-dihydroxypropan-2-iloxy)methyl)-2-amine-1H-purin-6(9H)-one is suspended in demineralized water, under strong stirring, under room temperature until complete homogenization.

The proportion of demineralized water used in relation to 9-((1,3-dihydroxypropan-2-iloxy)methyl)-2-amine-1H-purin-6(9H)-one is preferably 10 parts, although it is possible to use from 8 to 20 parts achieving the same effect.

Under stirring, sodium hydroxide (caustic soda) is added in an equivalent amount of 1.1 mol of sodium hydroxide in relation to 9-((1,3-dihydroxypropan-2-iloxy)methyl)-2-amine-1H-purin-6(9H)-one, there is the total dissolution of the suspension. It is possible to use 0.9 to 2.0 moles of sodium hydroxide per mole of the 9-((1,3-dihydroxypropan-2-iloxy)methyl)-2-amine-1H-purin-6(9H)-one, preferably 1.1 moles.

Other inorganic bases that can be used are: potassium hydroxide and lithium hydroxide. Preferably sodium hydroxide is used. After that, under stirring, the temperature of the solution is raised to 75 to 90°C, preferably 85°C, and fuming hydrochloric acid (or other acid like hydrofluoric, acetic, citric, preferably hydrochloric acid is used) is added until the solution reaches a pH ranging from 4.5 to 5.5, preferably 4.5, by using approximately 5.4g to 6.6g, preferably 6g of hydrochloric acid.

After adjusting the solution pH the solution is cooled under stirring to a temperature of 5 to 7°C, preferably to a temperature of 5°C, in order to crystallize the 9-((1,3-dihydroxypropan-2-iloxy)methyl)-2-amine-1H-purin-6(9H)-one. The system is kept under stirring under this temperature for a period of time of 25 to 40 minutes, preferably for 30 minutes and then the suspension is filtered, washing the solid with water at a temperature of 5 to 7°C, in a ratio of 1/10 of the water volume used in the beginning of the process, and then the solid is washed with isopropanol kept at a temperature from 5 to 7°C in a ratio of 1/10 of the water volume used in the beginning of the process. It is possible to use other organic solvents instead isopropanol, like: acetone, ethanol, methanol. Preferably isopropanol is used. The resulting solid of 9-((1,3-dihydroxypropan-2-iloxy)methyl)-2-amine-1H-purin-6(9H)-one is transferred to a glass lined reactor with a reflux condenser and isopropanol is added in a ratio of 4 to 6 parts in relation to the solid mass of 9-((1,3-dihydroxypropan-2-iloxy)methyl)-2-amine-1H-purin-6(9H)-one, preferably using 4 parts, and under stirring this suspension is heated until refluxing. Other organic solvents can be used instead isopropanol, like: acetone, ethanol, methanol. Preferably isopropanol is used. The system is kept under reflux for 3 to 4 hours. The suspension is cooled to a temperature from 20 to 30°C and it is filtered. The resulting solid of 9-((1,3-dihydroxypropan-2-iloxy)methyl)-2-amine-1H-purin-6(9H)-one is dried in a vacuum oven during 3 to 4 hours at a temperature from 60 to 80°C. The final yield of the procedure is between 89 to 98%.

The objective of the present invention is the injectable solution of 9-((1,3-dihydroxypropan-2-iloxy)methyl)-2-amine-1H-purin-6(9H)-one in its elemental form (free acid form, non saline) that can be prepared as a glucose 5% solution or as a sodium chloride 0,9% solution, presenting as characteristics the fact of being stable for a period of time of at least 24 months, presenting a pH ranging from about 3.0 to about 7.5, more preferably ranging from about 3.2 to about 7.0, and can be sterilized by using high temperatures techniques, for instance by autoclaving, because it does not degrade under this condition.

For the injectable solution of 9-((1,3-dihydroxypropan-2-iloxy)methyl)-2-amine-1H-purin-6(9H)-one prepared in glucose solution the ideal pH ranges from about 3.0 to about 7.0, more preferably the pH ranges from about 3.2 to about 6.5. For the injectable solution prepared in sodium chloride 0.9% the ideal pH ranges from about 4.0 to about 7.5, more preferably the pH ranges from about 4.5 to about 7.0.

It is also an objective of the present invention a kind of packing suitable for packing the injectable solution in a closed system, which consists of a flexible plastic bag manufactured with tri-laminated material.

The flexible bag used in the closed system with the new 9-((1,3-dihydroxypropan-2-iloxy)methyl)-2-amine-1H-purin-6(9H)-one formulation is made by a film composed by three distinct layers, each one of them with a singular protection function. The process for producing the film is called coextrusion, where the layers are grouped together forming a single sheet. The outer layer is made of polyester, a heat resistant material, with optimum transparency and optimum resistance to abrasion and mechanical stress. The intermediate layer is made of polyethylene giving excellent flexibility and, because its intrinsic properties, works as a barrier against moisture and vapors exchange with the environment. The inner layer is made of propylene copolymer that is impermeable and posses excellent flexibility.; its main characteristic is that it is chemically inert and do not interact with the product filled within. All this characteristics make the tri-laminated the excellence packing when compared to PVC, which besides presenting plastifying and additive substances interacts with the product stored for longer periods of time.

Due the combination of the solution packed within this closed system, another objective of the present invention is a pharmaceutical presentation consisting of an injectable solution of 9-((1,3-dihydroxypropan-2-iloxy)methyl)-2-amine-1H-purin-6(9H)-one, diluted in glucose 5% or sodium chloride 0.9% solution packed in the closed system consisting of a tri-laminated material flexible bag, which is ready for the therapeutic administration of the product.

Until the present moment, the injectable pharmaceutical presentation available of 9-((1,3-dihydroxypropan-2-iloxy)methyl)-2-amine-1H-purin-6(9H)-one consists of a lyophilized powder within a vial, which have to be prepared immediately before the therapeutic administration because stability problems resulting from its elevate pH incompatible with diluents like glucose 5% solution or sodium chloride 0,9% solution.

The present invention solves this problem from the state of art by a formulation prepared in an acid pH, that can be directly obtained by the process for treating the crystals of 9-((1,3-dihydroxypropan-2-iloxy)methyl)-2-amine-1H-purin-6(9H)-one by the crystallization in an acid pH as described in the process for eliminating the alkaline residuals.

The crystal of 9-((1,3-dihydroxypropan-2-iloxy)methyl)-2-amine-1H-purin-6(9H)-one resulting from the process presents ideal conditions for being added to sodium chloride 0.9 or glucose solution without presenting degradation.

The earlier tests were tried with the 9-((1,3-dihydroxypropan-2-iloxy)methyl)-2-amine-1H-purin-6(9H)-one molecule used for preparing the lyophilized product. As result, there was obtained a yellowish and oxidized solution because of the high pH, that did not stabilize the glucose solution.

By altering the crystallization molecule of sodium Ganciclovir previously achieved by LABOGEN, a clear and non-oxidized solution with pH of about 5.5 was achieved. Stabilizing the molecule in this pH, allowed lowering hazards during manipulation, once the previous solution was too much alkaline (pH=11), and was too much irritating after its reconstitution being not possible to be in contact with the skin, mucous membranes and eyes. Using appropriate glasses and gloves were necessary when manipulating this substance.

In accordance with protocols from the American Society of Hospital Pharmacy - ASHP, Ganciclovir must be manipulated and prepared inside laminar flow chambers, thus preventing the contamination of the product by microorganisms and protecting the person and the environment from potential risks of the medicament. The appropriate equipment carrying this operation is a class II vertical laminar flow chamber BSC (bio-security chamber).

As the solution of the present invention is pre-diluted ready for the administration to a patient, manipulating steps by the nursing personnel from the hospital are minimum, the risks of contamination and the security of the operator are preserved. The product from the present invention in the closed system will eliminate the need of acquiring laminar flow equipments for preparing the medicament.

The pH of the 9-((1,3-dihydroxypropan-2-iloxy)methyl)-2-amine-1H-purin-6(9H)-one as a solution in a closed system was significant altered, making its manipulation easier, increasing the security to the operator, lowering contamination risks and expenses regarding equipments, specific areas and employees for its manipulation. The toxicity risk, like carcinogenicity and mutagenicity for the operator was eliminated, once the product is ready for administration.

By altering the pH, the solution does not provoke abscess or phlebitis, risks existing in the previous formulation (vial with lyophilized powder - ROCHE).

With the new presentation in a closed system, it is possible to observe that one step of the process was facilitated. Considering the particle size distribution of the starting material, the grinding step required for the production of the lyophilized powder can be eliminated, as during the process for manipulating 9-((1,3-dihydroxypropan-2-iloxy)methyl)-2-amine-1H-purin-6(9H)-one in solutions it is used high stirring reactors that promote an efficient dissolution assured by particle counting tests.

Laboratory tests performed were active pharmaceutical ingredient assay (ganciclovir) by HPLC, diluents analysis (sodium chloride and glucose) by photometry and polarimetry, pH potentiometric determination, particle counting, accelerated stability tests, search for degradation products by spectrophotometry (hydroxymethyl furfural) and microbiological analysis. *In vivo,* pyrogen testing in rabbits.

During the production (purification/crystallization)of 9-((1,3-dihydroxypropan-2-iloxy)methyl)-2-amine-1H-purin-6(9H)-one (free acid form), crystals are generated with the inclusion of some parts per billion of alkaline residues. These alkaline residues are responsible for glucose degradation and therefore provoke the degradation of 9-((1,3-dihydroxypropan -2-iloxy) methyl)-2- amine-1H-purin-6(9H)-one present in the glucose solution. The answer found was to eliminate the residue, searching for a new crystallization way, in acidic pH, and so, free from alkaline residues and, consequently, a new crystal form was obtained, which results demonstrate a great improvement in the stability of the product in glucose and sodium chloride solutions with 9-((1,3-dihydroxypropan-2-iloxy)methyl)-2-amine-1H-purin-6(9H)-one.

According to the process for eliminating alkaline residues described before, the active pharmaceutical ingredient 9-((1,3-dihydroxypropan-2-iloxy)methyl)-2-amine-1H-purin-6(9H)-one is prepared in its elemental form (free acid form), without free alkaline residues, a primordial issue for preparing the new product presentation, as a sodium chloride 0.9% or glucose solution ready for its administration.

Studies demonstrate that free alkaline residues causes glucose degradation, a starting material present in the formulation of 9-((1,3-dihydroxypropan-2-iloxy)methyl)-2-amine-1H-purin-6(9H)-one product, forming furfural and mehtylfurfural that react with 9-((1,3-dihydroxypropan-2-iloxy) methyl) -2-amine -1H-purin-6(9H)-one originating further substances still under study.

During long term stability study, hydroxyethyl furfural, which is a glucose degradation product, was monitored in Ganciclovir in glucose solution. Tests were performed at the beginning, at 12 months and 24 months. The presence of hydroxyethyl furfural comes from glucose degradation when submitted to outside specified pH limits and sterilization beyond time and temperature specified limits.

With this new pharmaceutical presentation, other medicine associations can be easily done, which could not be possible with the initial formulation because its strong alkaline pH: Doxorubicin HCl, Etoposide phosphate, Fluconazole, Sodium Methotrexate, Sargramostim and Thiotepa, in ordinary usual dosages.

Considering that the therapeutic dosage of the lyophilized reconstituted product is 5mg/kg by intravenous infusion every 12 hours during 14 to 21 days, we need the results from the clinical trials in order to delineate necessary adjustments of the therapeutic method for the inventive product stored in a closed system.

The following examples are illustrative but not exhaustive about the several possibilities resulting from the present invention.

### EXAMPLE I:

### PROCESS FOR PREPARING 9-((1,3-DIHYDROXYPROPAN-2-ILOXY) METHYL)-2-AMINE-1H-PURIN-6(9H)-ONE FREE ACID FORM IN ITS ELEMENTAL FORM, WITHOUT FREE ALKALINE RESIDUES

To a suspension of 100g of 9-((1,3-dihydroxypropan-2-iloxy)methyl)-2-amine-1H-purin-6(9H)-one in 1L of demineralized water add 15g of caustic soda, pH 11.5, total dissolution takes place. After that, raise the temperature of the solution to 85°C and add about 6g of fuming hydrochloric acid until pH = 4,5. The solution is cooled to 5°C and 9-((1,3-dihydroxypropan-2-iloxy)methyl)-2-amine-1H-purin-6(9H)-one (free acid form) crystallizes. After 30 minutes under stirring at 5°C, the solid is filtered and washed with isopropanol. The solid of 9-((1,3-dihydroxypropan-2-iloxy)methyl)-2-amine-1H-purin-6(9H)-one (free acid form) is suspended in isopropanol, under intense reflux, for 4 hours. The suspension is cooled to room temperature (25°C) and is immediately filtered. The resulting solid of 9-((1,3- dihydroxypropan -2-iloxy) methyl)-2-amine-1H-purin-6(9H)-one (free acid form) is dried in vacuum oven for 4 hours at a temperature of 70°C, yielding 95.4g of the dried compound.

### EXAMPLE II

### PROCESS FOR PREPARING 9-((1,3- DIHYDROXYPROPAN-2-ILOXY) METHYL)-2-AMINE-1H-PURIN-6(9H)-ONE FREE ACID FORM IN ITS ELEMENTAL FORM, WITHOUT FREE ALKALINE RESIDUES

In a glass reactor equipped with a reflux condenser suspend the 9-((1,3-dihydroxypropan-2-iloxy)methyl)-2-amine-1H-purin-6(9H)-one in demineralized water, in a ratio of 10 parts of demineralized water in relation to 9-((1,3-dihydroxypropan-2-iloxy)methyl)-2-amine-1H-purin-6(9H)-one, under strong stirring at room temperature until complete homogenization. To the resulting suspension add under stirring sodium hydroxide (caustic soda), in an equivalent amount to 1.1 moles of sodium hydroxide in relation to 9-((1,3-dihydroxypropan -2-iloxy) methyl) -2-amine-1H-purin-6(9H)-one, total dissolution of the suspension takes place. After that, under stirring, raise the temperature of the solution to 85°C and add fuming hydrochloric acid until pH of the solution is 4.5, using about 5.4 to 6.6g of hydrochloric acid. After the solution pH adjustment, start its cooling under stirring until temperature reaches 5°C, in order to crystallize the 9-((1,3-dihydroxypropan-2-iloxy)methyl)-2-amine-1H-purin-6(9H)-one. The resulting suspension is kept under stirring at that temperature for 30 minutes and then it is filtered, the solids being washed with water kept at a temperature of 5 to 7°C in a ratio of 1/10 of the volume of water used in the beginning of the process and then it is washed with isopropanol kept under a temperature of 5 to 7°C in a ratio of 1/10 of the volume of water used in the beginning of the process. The resulting solid of 9-((1,3-dihydroxypropan-2-iloxy)methyl)-2-amine-1H-purin-6(9H)-one is transferred to a glass lined reactor equipped with a reflux condenser, isopropanol is added in a ratio of 4 parts in relation to the solid mass of 9-((1,3-dihydroxypropan-2-iloxy)methyl)-2-amine-1H-purin-6(9H)-one and, under stirring, this suspension is heated to reflux temperature. Reflux is kept for 3 to 4 hours. The suspension is cooled to a temperature between 20 and 30°C and filtered. The resulting solid of 9-((1,3-dihydroxypropan-2-iloxy)methyl)-2-amine-1H-purin-6(9H)-one is dried in vacuum oven for 3 to 4 hours at a temperature ranging from 60° to 80°C. The final yield is between 89 to 98%.

### TABLE 1: SATBILITY STUDY

Product: Ganciclovir in glucose 5% solution (1mg/mL)
Batch Number: Pilot 1 Manufacturing Date: Feb/2000
Batch size: 100 units
Packing: Tri-laminate plastic bag - 250mL
Results:

### Long term stability Study - 30°C ± 2°C:

| **Tests.** | **Specification** | **Beginning** | **6 months** | **9 months** | **12 months** | **24 months** |
|---|---|---|---|---|---|---|
| **Description/ color** | Clear colorless liquid | Conforms | Conforms | Conforms | Conforms | Conforms |
| **Assay** 90% to **Ganciclo-vir** | 110% | 101.2 | 100.8 | 99.5 | 99.7 | 98.7 |
| **Assay Glucose** | 95% to 105% | 99.8 | 99.2 | 99.4 | 98.8 | 98.0 |
| **pH** | 3.2 to 6.5 | 5.8 | 5.4 | 5.5 | 5.7 | 5.5 |
| **Sterility** | Sterile | Sterile | - | - | - | Sterile |
| **Pyrogen** | Non-pyrogenic | | - | - | - | Non-pyrogenic |
| **Number of analyzed samples** | | 13 units | 3 units | 3 units | 3 units | 13 units |

### Accelerated Stability:

The Injectable solution of Ganciclovir in glucose 5% was submitted in its primary packing to study in ovens at temperatures of 40°C and 50°C for periods of 180 and 90 days respectively. Bags placed at 40°C were analyzed in periods of 30, 60, 90 and 180 days, and bags placed at 50°C were analyzed in periods of 30, 60 and 90 days.

**TABLE II: STABILITY STUDY**

| **Tests** | | **Aspect: Clear colorless liquid** | **pH : 3.2 to 6.5** | **Glucose Assay: 95% to 105%** | **Ganciclovir Assay: 90% to 110%** | **Pyrogen: (USP 24) Sterility: Sterile (USP 24)** | **Pyrogen: Non-pyrogenic (USP 24)** | **Number of samples analyzed** |
|---|---|---|---|---|---|---|---|---|
| **Batch: Pilot 01** | | | | | | | | |
| 40°C | 30 days | Conforms | 5.6 | 99.8% | 101.2% | - | - | 3 units |
| | 60 days | Conforms | 5.8 | 99.6% | 100.7% | - | - | 3 units |
| | 90 days | Conforms | 5.2 | 99.1% | 99.7% | - | - | 3 units |
| | 180 days | Conforms | 5.5 | 98.7% | 99.0% | Sterile | Non-pyrogenic | 13 units |
| 50°C | 30 days | Conforms | 5.7 | 99.7% | 101.3% | - | - | 3 units |
| | 60 days | Conforms | 5.1 | 99.4 % | 100.8 % | - | - | 3 units |
| | 90 days | Conforms | 5.5 | 98.8% | 99.8% | Sterile | Non-pyrogenic | 13 units |

Product: Ganciclovir in glucose 5% solution (1mg/mL)
Batch Number: Pilot 2 Manufacturing Date: Feb/2000
Batch size: 100 units
Packing: Tri-laminate plastic bag - 250mL
Results:

### Long term stability Study - 30°C ± 2°C:

| **Tests** | **Specification** | **Beginning** | **6 months** | **9 months** | **12 months** | **24 months** |
|---|---|---|---|---|---|---|
| **Description/ color** | Clear colorless liquid | Conforms | Conforms | Conforms | Conforms | Conforms |
| **Ganciclovir** | 90% to 110% | 98.6% | 98.0% | 97.8% | 97.5% | 96.9% |
| **Assay Glucose** | 95% to 105% | 99.6% | 99.2% | 9 8.8 % | 98.0% | 97.8% |
| **pH** | 3.2 a 6.5 | 4.8 | 5.0 | 5 . 1 | 4.9 | 5.0 |
| **Sterility** | Sterile | Sterile | - | - | - | Sterile |
| **Pyrogen** | Non-pyrogenic | Non-pyrogenic | - | - | - | Non-pyrogenic |
| **Number of samples analyzed** | - | 13 units | 3 units | 3 units | 3 units | 13 units |

### Accelerated Stability:

The Injectable solution of Ganciclovir in glucose 5% was submitted in its primary packing to study in ovens at temperatures of 40°C and 50°C for periods of 180 and 90 days respectively. Bags placed at 40°C were analyzed in periods of 30, 60, 90 and 180 days, and bags placed at 50°C were analyzed in periods of 30, 60 and 90 days.

**TABLE III: STABILITY STUDY**

| **Tests** | | **Aspect: Clear colorless liquid** | **pH : 3.2 to 6.5** | **Glucose Assay: 95% to 105%** | **Ganciclovir Assay: 90% to 110%** | **Pyrogen: (USP 24) Sterility: Sterile (USP 24)** | **Pyrogen: Non-pyrogenic (USP 24)** | **Number of samples analyzed** |
|---|---|---|---|---|---|---|---|---|
| **Batch: Pilot 02** | | | | | | | | |
| 40°C | 30 days | Conforms | 4.8 | 98.6% | 99.6% | - | - | 3 units |
| | 60 days | Conforms | 5.1 | 98.2% | 99.0% | - | - | 3 units |
| | 90 days | Conforms | 5.0 | 97.7% | 98.7% | - | - | 3 units |
| | 180 days | Conforms | 5.3 | 97.2% | 98.0% | Sterile | Non-pyrogenic | 13 units |
| 50°C | 30 days | Conforms | 4.9 | 98.7% | 99.7% | - | - | 3 units |
| | 60 days | Conforms | 5.0 | 98.4% | 99.2% | - | - | 3 units |
| | 90 days | Conforms | 5.2 | 97.8% | 98.8% | Sterile | Non-pyrogenic | 13 units |

Product: Ganciclovir in glucose 5% solution (1mg/mL)
Batch Number: Pilot 3 Manufacturing Date: Feb/2000
Batch size: 100 units
Packing: Tri-laminate plastic bag - 250mL
Results:

### Long term stability Study - 30°C ± 2°C:

| **Tests** | **Specification** | **Beginning** | **6 months** | **9 months** | **12 months** | **24months** |
|---|---|---|---|---|---|---|
| **Description/ color** | **Clear colorless** | **Conforms** | **Conforms** | **Conforms** | **Conforms** | **Conforms** |
| **Assay Ganciclo-vir** | 90% to 110%: | 99.5% | 99.0% | 98.5% | 98.0% | 97.8% |
| **Assay Glucose** | 95% to 105% | 98.9% | 98.2% | 98.4% | 98.2% | 98.0% |
| **pH** | 3. 2 to 6 . 5 | 4. 8 | 5. 0 | 5.1 | 5.2 | 5.0 |
| **Sterility** | Sterile | Sterile | - | - | - | Sterile |
| **Pyrogen** | Non-pyrogenic | Non-pyrogenic | - | - | - | Non-pyrogenic |
| **Number of samples analyzed** | - | 13 units | 3 units | 3 units | 3 units | 13 units |

### Accelerated Stability:

The Injectable solution of Ganciclovir in glucose 5% was submitted in its primary packing to study in ovens at temperatures of 40°C and 50°C for periods of 180 and 90 days respectively. Bags placed at 40°C were analyzed in periods of 30, 60, 90 and 180 days, and bags placed at 50°C were analyzed in periods of 30, 60 and 90 days.

**TABLE IV: STABILITY STUDY**

| **Tests** | | **Aspect: Clear colorless liquid** | **pH : 3.2 to 6.5** | **Glucose Assay: 95% to 105%** | **Ganciclovir Assay: 90% to 110%** | **Pyrogen: (USP 24) Sterility: Sterile (USP 24)** | **Pyrogen: Non-pyrogenic (USP 24)** | **Number of samples analyzed** |
|---|---|---|---|---|---|---|---|---|
| **Batch: Pilot 02** | | | | | | | | |
| 40°C | 30 days | Conforms | 4.8 | 98.8% | 99.2% | - | - | 3 units |
| | 60 days | Conforms | 5.2 | 97.8% | 98.8% | - | - | 3 units |
| | 90 days | Conforms | 5.0 | 97.1% | 98.2% | - | - | 3 units |
| | 180 days | Conforms | 5.2 | 97.0% | 97.6% | Sterile | Non-pyrogenic | 13 units |
| 50°C | 30 days | Conforms | 4.9 | 98.7% | 99.4% | - | - | 3 units |
| | 60 days | Conforms | 5.1 | 98.4% | 99.0% | - | - | 3 units |
| | 90 days | Conforms | 5.0 | 97.8% | 98.8% | Sterile | Non-pyrogenic | 13 units |

Product: Ganciclovir in sodium chloride 0.9% solution (1mg/mL)
Batch Number: Pilot Manufacturing Date: Aug/2002
Batch size: 100 units
Packing: Tri-laminate plastic bag - 250mL
Results:

### Long term stability Study - 30°C ± 2°C:

| **Tests** | **Specification** | **Beginning** | **6 months** | **9 months** | **12 months** | **24 months** |
|---|---|---|---|---|---|---|
| **Description/ color** | **Clear colorless liquid** | Conforms | Conforms | Conforms | Conforms | Conforms |
| **Assay Ganciclo-vir** | **90% a 110%** | 98.20% | 96.70% | 92.66% | 95.20% | 95.00% |
| **Assay Sodium chloride (0.9%)** | **95% a 105%** | 100.00% | 100.50% | 100.00% | 99.80% | 99.50% |
| **pH** | **4.5 to 7.0** | 5.71 | 5.13 | 6.21 | 5.74 | 5.7 |
| **Sterility Sterile** | **Sterile** | Sterile | - | - | - | Sterile |
| **Pyrogen** | **Non-pyrogenic** | Non-pyrogenic | - | - | - | Non-pyrogenic. |
| **Number of samples analyzed** | | 13 units | 3 units | 3 units | 3 units | 13 units |

### Accelerated Stability:

The Injectable solution of Ganciclovir in sodium chloride 0.9%% was submitted in its primary packing to study in ovens at temperatures of 40°C and 50°C for periods of 180 and 90 days respectively. Bags placed at 40°C were analyzed in periods of 30, 60, 90 and 180 days, and bags placed at 50°C were analyzed in periods of 30, 60 and 90 days.

| **Tests** | | **Aspect: Clear colorless liquid** | **pH : 4.5 to 7.0** | **Assay NaCl 0.9%: 95% to 105%** | **Assay Ganciclovir 90% to 110%** | **Pyrogen: Non-pyrogenic (USP 24)** | **Sterility: Sterile (USP 24)** | **Number of samples analyzed** |
|---|---|---|---|---|---|---|---|---|
| **Batch: Pilot** | | | | | | | | |
| 40°C | 30 days | Conforms | 5.46 | 98.30% | 100.00% | - | - | 3 units |
| | 60 days | Conforms | 5.59 | 98.00% | 101.20% | - | - | 3 units |
| | 90 days | Conforms | 5.30 | 97.50% | 100.50% | - | - | 3 units |
| | 180 days | Conforms | 5.32 | 97.00% | 99.80% | Non-pyrogenic | Sterile | 13 units |
| 50°C | 30 days | Conforms | 5.33 | 99.92% | 100.60% | - | - | 3 units |
| | 60 days | Conforms | 5.48 | 98.30% | 100.60% | - | - | 3 units |
| | 90 days | Conforms | 5.29 | 97.50% | 99.5% | Non-pyrogenic | Sterile | 13 units |

Only as illustrating, as described in literature related to the issue (Guideline for Parenteral Administration of Antimicrobials F. Hoffmann - La Roche Ltd, Basel - Switzerland) the following recommendations are necessary for administration of Ganciclovir as described in the state of art, evidencing the profoundness of the inventive activity of the present invention, which advantages over the state of art are evident:

### RECOMMENDATION

Reconstitute each vial with 10mL of sterile water for injection and dilute in an acceptable volume of infusion solution, administrating it by intravenous infusion for a period of approximately one hour.

### BOLUS INTRAVENOUS

No. Toxicity may increase.

### INTERMITTENT PERFUSION

Yes. Dilute the reconstituted content in an infusion solution and deliver it over the course of one hour.

### CONTINUOUS INTRAVENOUS

Do not recommended due the risk of bacterial contamination.

### INTRAMUSCULAR

Yes, but can cause irritation in the site of the injection due the elevate pH of the injectable solution (pH ~ 11). It also can be done by subcutaneous administration, but with the same local irritation risk.

### COMPATIBLE SOLUTIONS

Sodium chloride 0.9% aqueous solution.
Glucose 5% aqueous solution.
Ringer solution.
Ringer lactated solution.

### STABILITY

12 hours under room temperature when reconstituted inside its own vial.

When diluted ,use it as soon as possible because the risk of bacterial contamination. If it is not possible to use it immediately, keep it under refrigeration for a maximum period of 24 hours, avoiding freezing.

### OBSERVATIONS

Do not dilute with bacteriostatic water. Only use sterile bi-distilled water for injection.

Inspect the vial after reconstitution to avoid presence of particulate matter or discoloration.

Not less important is described, also as illustrative matter, in the Technical Opinion n° 005/95 from Conselho Federal de Farmacia - Brazil, about the Validation of Technical Analysis Appropriate to Products and By-products Resulting from obtaining the New Developed Molecule.

Subject: Preparing and administering intravenous Ganciclovir and Anphotericin B.

### Analysis

Considering the solicitation issued in the Conselho Federal de Farmacia that forwarded the following informations:
Ganciclovir, an antiviral agent, is considered a risk drug because its carcinogenic and mutagenic properties. So, care must be taken when manipulating, preparing and administering it.

Because the resulting solution is strongly alkaline (pH=11) and irritating after its reconstitution, one must avoid the contact with the skin, mucous membranes and the eyes. Using glasses and gloves are necessary when manipulating it.

In accordance with protocols from the American Society of Hospital Pharmacy - ASHP, Ganciclovir must be manipulated and prepared inside laminar flow chambers, preventing the contamination of the product by microorganisms and protecting the individual and the environment from potential risks of the medicament. The appropriate equipment for carrying this operation is a class II vertical laminar flow chamber BSC (bio-security chamber).

Initially, Amphotericin B (a fungicide) must be reconstituted with 10mL of sterile water for injection, without bacteriostatic agent, for achieving a concentration - of 5mg/mL, forming a colloidal suspension. For the intravenous infusion, the colloidal suspension must be diluted to 500mL with glucose 5% solution, reaching a concentration of 0.1mg/mL.

Solutions containing electrolytes (for instance NaCl 0.9%) or containing bacteriostatic agents should not be used for reconstituting and/or diluting Amphotericin B, because there is the risk of precipitating the drug.

Amphotericin B, for administration by intravenous infusion must be prepared according to rigorous aseptic techniques, it means, using sterile gloves, needles and syringes, by cleaning the vial top with a cotton soaked in alcohol, etc.

Although Amphotericin B is sensible to light, it is not necessary to cover the infusion bag, if the administration takes place within until 8 hours after its preparation.

Considering that new drugs are being launched for treating immunodepressed patients everyday and it is very much usual the lack of knowledge of the health professional personnel about the risks that they could be exposed to when preparing and administering these drugs, it is necessary for them to keep under a constant upgrading program.

## Claims

1. Process of making crystals of 9-((1,3-DIHYDROXYPROPAN-2-YLOXY) METHYL)-2-AMINO-1H-PURIN1-6-(9H)-ONE, free from alkaline residues **characterized by** comprising the following steps:
a) Suspending the 9-((1,3-DIXYDROXYPROPAN-2-YLOXY)METHYL)-2-AMINO-1H-PURIN-6-(9H),ONE in demineralized water;
b) Elevating the pH to a range between 10.5 and 12.5 by adding inorganic bases;
c) Elevating the temperature of the resulting solution 1 (b) to a range between 75° and 90°C;
d) Adding inorganic or organic acids adjusting the pH in a range from 4.5 to 5.5;
e) Cooling the solution to a temperature ranging from 5° to 7°C and keeping the resulting crystals of 9-((1,3-DINYDROXYPROPAN-2-YLOXY)METHYL)-2-AMINO-1H-PURIN-6-(9H)-ONE under stirring for 25 to 40 minutes;
f) Filtering the resulting crystals from 1(e) and washing with an organic solvent selected from the group comprising acetone, ethanol, methanol and isopropanol;
g) Intense refluxing the resulting crystals from 1(f) in an organic solvent selected from the group comprising methanol, ethanol, propanol, isopropanol and butanol, for a period of time ranging from 3 to 4 hours;
h) Cooling the resulting suspension from 1(g) to a temperature ranging from 20° and 30°C, filtering the crystals and drying them under vacuum and under a temperature ranging from 60° and BO°C.

2. Process according to claim 1, **characterized by** the inorganic base used in 1(b) being selected from the group comprising potassium hydroxide, lithium hydroxide and sodium hydroxide.

3. Process according to claim 2, **characterized by** the inorganic base is sodium hydroxide.

4. Process according to claim 1, **characterized by** the organic solvent used in steps 1(f) and 1(g) is isopropanol.

5. A ready-for-use pharmaceutical formulation sterile, stable, in closed system, **characterized by** comprising an injectable aqueous solution of crystals from active principle 9- ((1,3-DIHYDROXYPROPAN-2-YLOXY)METHYL)-2-MINO-1H-PURIN-6-(9H)-ONE as its free acid form, prepared according to process of claim 1, diluted in glucose 5% solution or sodium chloride 0.9% solution, with pH ranging from 3.0 to 6.9, and being packed in a special packing, which is a flexible bag manufactured with a tri-laminated material composed by three distinct layers, being an external layer of polyester, an intermediate layer of polyethylene and the inner layer of propylene copolymer.

6. Pharmaceutical formulation according to claim 5, **characterized by** the solution is a sodium chloride 0.9% solution, the pH is within the range of 4.5 to 6.9.

7. Pharmaceutical formulation according to claim 5, **characterized by** the solution is a glucose 5% solution, the pH is within the range of 3.2 to 6.5.

8. Use of an inert closed system **characterized by** comprising a flexible bag manufactured by a tri-laminated material coextrused, composed by three distinct layers, being an external anti-thermic and clear layer of polyester, an intermediate barrier layer of polyethylene and the inner inert layer of propylene copolymer for packing glucose 5% or sodium chloride 0.9% aqueous solutions comprising 9-((1,3-DIHYDROXYPROPAN-2-YLOXY)MExHYL)-2-AMINO-1H-PURIN-6-(9H)-ONE crystals free from alkaline residues prepared according to claim 1.

## Patentansprüche

1. Verfahren zur Herstellung von Kristallen von 9-((1,3-DIHYDROXYPROPAN-2-YLOXY)METHYL)-2-AMINO-1H-PURIN-6-(9H)-ON frei von alkalischen Rückständen, **dadurch gekennzeichnet, daß** es die folgenden Schritte umfaßt:
a) Suspendieren des 9-((1,3-DIHYDROXYPROPAN-2-YLOXY)METHYL)-2-AMINO-1H-PURIN-6-(9H)-ONs in vollentsalztem Wasser;
b) Erhöhen des pH-Wertes auf einen Bereich zwischen 10,5 und 12,5 durch Zugabe anorganischer Basen;
c) Erhöhen der Temperatur der auf diese Weise erhaltenen Lösung 1(b) auf einen Bereich zwischen 75°C und 90°C .
d) Zugabe von anorganischen oder organischen Säuren zum Einstellen des pH-Wertes auf einen Bereich von 4,5 bis 5,5;
e) Kühlen der Lösung auf eine Temperatur im Bereich von 5°C bis 7°C, wobei die auf diese Weise erhaltenen Kristalle von 9-((1,3-DIHYDROXYPROPAN-2-YLOXY)METHYL)-2-AMINO-1H-PURIN-6-(9H)-ON 25 bis 40 Minuten lang am Rühren gehalten werden;
f) Filtrieren der auf diese Weise erhaltenen Kristalle aus 1(e) und Waschen mit einem organischen Lösungsmittel ausgewählt aus der Aceton, Ethanol, Methanol und Isopropanol umfassenden Gruppe;
g) Intensives Erhitzen der auf diese Weise erhaltenen Kristalle von 1(f) unter Rückfluß in einem organischen Lösungsmittel ausgewählt aus der Methanol, Ethanol, Propanol, Isopropanol und Butanol umfassenden Gruppe, über eine Zeitspanne im Bereich von 3 bis 4 Stunden;
h) Abkühlen der auf diese Weise erhaltenen Suspension von 1(g) auf eine Temperatur im Bereich von 20°C bis 30°C, Filtrieren und Trocknen der Kristalle im Vakuum bei einer Temperatur im Bereich von 60°C bis 80°C.

2. Verfahren nach Anspruch 1, **dadurch gekennzeichnet, daß** die in 1(b) verwendete anorganische Base aus der Kaliumhydroxid, Lithiumhydroxid und Natriumhydroxid umfassenden Gruppe ausgewählt ist.

3. Verfahren nach Anspruch 2, **dadurch gekennzeichnet, daß** es sich bei der anorganischen Base um Natriumhydroxid handelt.

4. Verfahren nach Anspruch 1, **dadurch gekennzeichnet, daß** es sich bei dem in den Schritten 1(f) und 1(g) verwendeten organischen Lösungsmittel um Isopropanol handelt.

5. Gebrauchsfertige, sterile, stabile pharmazeutische Formulierung in einem geschlossenen System, **dadurch gekennzeichnet, daß** sie eine wäßrige Injektionslösung von Kristallen des Wirkstoffs 9-((1,3-DIHYDROXYPROPAN-2-YLOXY)METHYL)-2-AMINO-1H-PURIN-6-(9H)-ON in Form dessen freier Säure, hergestellt gemäß dem Verfahren von Anspruch 1, verdünnt mit 5%iger Glucoselösung oder 0,9%iger Natriumchloridlösung mit einem pH-Wert im Bereich von 3,0 bis 6,9 enthält und in einer speziellen Verpackung verpackt ist, bei der es sich um einen flexiblen Beutel handelt, der aus einem dreifach laminierten Material hergestellt ist, das aus drei unterschiedlichen Lagen besteht, bei denen es sich um eine äußere Polyesterlage, eine Polyethylenzwischenlage und eine innere Propylencopolymerlage handelt.

6. Pharmazeutische Formulierung nach Anspruch 5, **dadurch gekennzeichnet, daß** es sich bei der Lösung um eine 0,9%ige Natriumchloridlösung mit einem pH-Wert im Bereich von 4,5 bis 6,9 handelt.

7. Pharmazeutische Formulierung nach Anspruch 5, **dadurch gekennzeichnet, daß** es sich bei der Lösung um eine 5%ige Glucoselösung mit einem pH-Wert im Bereich von 3,2 bis 6,5 handelt.

8. Verwendung eines inerten geschlossenen Systems, **dadurch gekennzeichnet, daß** es einen flexiblen Beutel, hergestellt aus einem aus drei unterschiedlichen Lagen, bei denen es sich um eine äußere antithermische und durchsichtige Polyesterlage, eine als Barriere wirkende Polyethylenzwischenlage und eine inerte innere Propylencopolymerlage handelt, bestehenden coextruierten, dreifach laminierten Material, umfaßt, zur Verpackung von wäßrigen 5%igen Glucose- oder 0,9%igen Natriumchloridlösungen, die nach Anspruch 1 hergestellte 9-((1,3-DIHYDROXYPROPAN-2-YLOXY)METHYL)-2-AMINO-1H-PURIN-6-(9H)-ON-Kristalle frei von alkalischen Rückständen enthalten.

## Revendications

1. Procédé de préparation de cristaux de 9-((1,3-dihydroxypropan-2-yloxy)méthyl)-2-amino-1H-purin-6-(9H)-one, exempts de résidus alcalins **caractérisé en ce qu'**il comprend les étapes suivantes consistant à :
a) mettre en suspension la 9-((1,3-dihydroxypropan-2-yloxy)méthyl)-2-amino-1H-purin-6-(9H)-one dans de l'eau déminéralisée ;
b) augmenter le pH dans une plage comprise entre 10,5 et 12,5 en ajoutant des bases inorganiques ;
c) augmenter la température de la solution résultante 1(b) dans une plage comprise entre 75 °C et 90 °C ;
d) ajouter des acides inorganiques ou organiques de manière à ajuster le pH dans une plage de 4,5 à 5,5 ;
e) refroidir la solution à une température dans la plage de 5 °C à 7 °C et maintenir les cristaux résultants de 9-((1,3-dihydroxypropan-2-yloxy)méthyl)-2-amino-1H-purin-6-(9H)-one sous agitation pendant 25 à 40 minutes ;
f) filtrer les cristaux résultants de 1(e) et laver avec un solvant organique choisi dans le groupe constitué de l'acétone, l'éthanol, le méthanol et l'isopropanol ;
g) chauffer à reflux intense les cristaux résultants de 1(f) dans un solvant organique choisi dans le groupe constitué du méthanol, de l'éthanol, du propanol, de l'isopropanol et du butanol, pendant une durée dans la plage de 3 à 4 heures ;
h) refroidir la suspension résultante de 1(g) à une température dans la plage de 20 °C à 30 °C, filtrer les cristaux et sécher ceux-ci sous vide et à une température dans la plage de 60 °C à 80 °C.

2. Procédé selon la revendication 1, **caractérisé en ce que** la base inorganique utilisée dans 1(b) est choisie dans le groupe constitué de l'hydroxyde de potassium, l'hydroxyde de lithium et l'hydroxyde de sodium.

3. Procédé selon la revendication 2, **caractérisé en ce que** la base inorganique est l'hydroxyde de sodium.

4. Procédé selon la revendication 1, **caractérisé en ce que** le solvant organique utilisé dans les étapes 1(f) et 1(g) est l'isopropanol.

5. Formulation pharmaceutique stérile prête à l'usage, stable, en système fermé, **caractérisée en ce qu'**elle comprend une solution aqueuse injectable de cristaux du principe actif 9-((1,3-dihydroxypropan-2-yloxy)méthyl)-2-amino-1H-purin-6-(9H)-one sous sa forme acide libre, préparée selon le procédé de la revendication 1, diluée dans une solution de glucose à 5 % ou une solution de chlorure de sodium à 0,9 %, ayant un pH dans la plage de 3,0 à 6,9, et étant conditionnée dans un emballage spécial, qui est une poche flexible fabriquée avec un matériau trilaminé composé de trois couches distinctes, à savoir une couche externe de polyester, une couche intermédiaire de polyéthylène et une couche interne de copolymère de propylène.

6. Formulation pharmaceutique selon la revendication 5, **caractérisée en ce que** la solution est une solution de chlorure de sodium à 0,9 %, le pH est dans la plage de 4,5 à 6, 9 .

7. Formulation pharmaceutique selon la revendication 5, **caractérisée en ce que** la solution est une solution de glucose à 5 %, le pH est dans la plage de 3,2 à 6,5.

8. Utilisation d'un système inerte **caractérisé en ce qu'**il comprend une poche flexible fabriquée avec un matériau trilaminé coextrudé, composé de trois couches distinctes, à savoir une couche externe antithermique et transparente de polyester, une couche de barrière intermédiaire de polyéthylène et la couche inerte interne de copolymère de propylène pour conditionner des solutions aqueuses de glucose à 5 % ou de chlorure de sodium à 0,9 % comprenant des cristaux de 9-((1,3-dihydroxypropan-2-yloxy)méthyl)-2-amino-1H-purin-6-(9H)-one exempts de résidus alcalins préparés selon la revendication 1.
